(19) Europäisches Patentamt
European Patent Office
Office européen des brevets

(11) Veröffentlichungsnummer: **0 199 982 B1**

(12) **EUROPÄISCHE PATENTSCHRIFT**

(45) Veröffentlichungstag der Patentschrift: **03.04.91**

(51) Int. Cl.5: **C07D 233/56**, C07D 249/08, C07D 405/06, A01N 43/30, A01N 43/653

(21) Anmeldenummer: **86103971.7**

(22) Anmeldetag: **22.03.86**

Die Akte enthält technische Angaben, die nach dem Eingang der Anmeldung eingereicht wurden und die nicht in dieser Patentschrift enthalten sind.

(54) **Azolverbindungen, Verfahren zu ihrer Herstellung und Mittel zur Regulierung des Pflanzenwachstums.**

(30) Priorität: **01.04.85 DE 3511922**

(43) Veröffentlichungstag der Anmeldung: **10.12.86 Patentblatt 86/45**

(45) Bekanntmachung des Hinweises auf die Patenterteilung: **03.04.91 Patentblatt 91/14**

(84) Benannte Vertragsstaaten: **AT BE CH DE FR GB IT LI NL SE**

(56) Entgegenhaltungen:
**EP-A- 0 079 006**
**EP-A- 0 113 839**
**FR-A- 2 346 338**
**FR-A- 2 379 250**
**GB-A- 2 003 848**

(73) Patentinhaber: **BASF Aktiengesellschaft**
**Carl-Bosch-Strasse 38**
**W-6700 Ludwigshafen(DE)**

(72) Erfinder: **Tuerk, Wolfgang, Dr.**
**Wittelsbachstrasse 61**
**W-6700 Ludwigshafen(DE)**
Erfinder: **Rentzea, Costin, Dr.**
**Richard-Kuhn-Strasse 1-3**
**W-6900 Heidelberg(DE)**
Erfinder: **Sauter, Hubert, Dr.**
**Neckarpromenade 20**
**W-6800 Mannheim 1(DE)**
Erfinder: **Martin, Roland, Dr.**
**Bruesseler Ring 53**
**W-6700 Ludwigshafen(DE)**
Erfinder: **Rademacher, Wilhelm, Dr.**
**Austrasse 1**
**W-6703 Limburgerhof(DE)**
Erfinder: **Jung, Johann, Prof. Dr.**
**Hardenburgstrasse 19**
**W-6703 Limburgerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

·Die vorliegende Erfindung betrifft neue Azolverbindungen, Verfahren zu ihrer Herstellung, diese Verbindungen enthaltende Mittel und ihre Verwendung als Wachstumsregulatoren.

Es ist bekannt, 2-Chlorethyl-trimethylammoniumchlorid (Chlorcholinchlorid, CCC; vgl. US-A 3 156 554) zur Beeinflussung des Pflanzenwachstums zu verwenden. Mit seiner Hilfe läßt sich z. B. eine Hemzung des Längenwachstums bei einigen Getreidearten und eine Hemmung des vegetativen Wachsts bei einigen anderen Kulturpflanzen erreichen. Die Wirkung dieser Substanz ist jedoch bei manchen Arten und bei niedrigen Aufwandmengen nicht immer ausreichend.

Man hat daher nach anderen Wirkstoffen vergleichbarer Wirkungsrichtung gesucht; so ist z.B. bekannt, 3,3-Dimethyl-2-(1,2,4-triazol-1-yl)-1-(4-chlorbenzoyl)-butan zur Beeinflussung des Pflanzenwachstums zu verwenden (DE-A 27 39 352). Die Wirkung ist jedoch bei niedrigen Aufwandmengen und Aufwandkonzentrationen nicht immer befriedigend; auch ist die wachstumsregulierende Wirkung oft mit einer hohen Phytotoxizität verbunden, so daß in den für eine wachstumsregulierende Wirkung nötigen Konzentrationen auch eine Schädigung der Kulturpflanzen beobachtet wird. Außerdem sind Azolylderivate mit wachstumsregulierender und/oder fungizider Wirkung bekannt (DE-A 2 805 277, DE-A 2 638 470, EP-A 113 839, EP-A 79 006).

Es wurden nun neue Verbindungen gefunden, die eine hohe wachstumsregulierende Wirkung bei guter Pflanzenverträglichkeit aufweisen, und zwar Verbindungen der Formel I

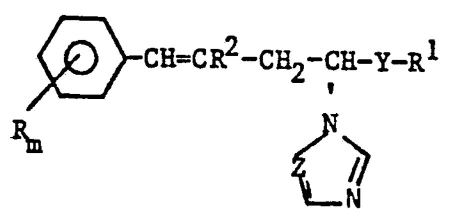

(I),

in welcher

R Fluor, Ghlor, Brom, $C_1$-$C_4$-Alkyl, $C_1$-$C_4$-Alkoxy, Trifluormethyl, Phenyl oder Phenoxy beaeutet und m = 0 oder eine ganze Zahl von 1 bis 5 und wobei die einzelnen Atome oder Gruppen gleich oder verschieden sein können, wenn m größer als 1 ist,

$R^1$ tert.-Butyl, 5-Methyl-1,3-dioxan-5-yl, 1-Methylcyclohexyl, 2-Methyltetrahydropyran-2-yl oder

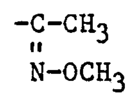

,

$R^2$ Wasserstoff oder $C_1$-$C_5$-Alkyl, ferner

Y $>C=O$, $>C=N$-OH oder $>CR^3OR^4$ bedeutet, mit der Maßgabe, daß nicht gleichzeitig Y = $>C=O$, $R^1$ = tert.-Butyl und m = O ist, und

Z $>CH$ oder $>N$ ist, wobei

$R^3$ Wasserstoff, Methyl, Vinyl, Allyl, Ethinyl oder Propinyl und

$R^4$ Wasserstoff, $C_1$-$C_4$-Alkyl, $C_2$-$C_4$-Alkenyl, $C_2$-$C_4$-Alkinyl oder $C_2$-$C_4$-Alkanoyl bedeutet.

Sie werden als solche oder in Form ihrer für Pflanzen verträglichen Säureadditionssalze und Metallkomplexe als Wirkstoffe mit sehr guter wachstumsregulierender Wirkung bei ausgezeichneter Verträglichkeit verwendet.

In den Verbindungen der Formel I sind das azolylsubstituierte Kohlenstoffatom und das benachbarte, durch Sauerstoff substituierte Kohlenstoffatom chiral; die Doppelbindung kann daher cis- oder trans-konfiguriert sein; die Wirkstoffe fallen demgemäß als Enantiomerengemische an, die in die optisch aktiven Enantiomeren getrennt werden können, soweit nicht isomerenreine Vorprodukte verwendet worden sind. Im allgemeinen fallen die Wirkstoffe bedingt durch die Anwesenheit zweier chiraler Zentren auch als Diastereomerengemische an, die in üblicher Weise, z. B. durch Kristallisation oder Chromatographie in die einzelnen Komponenten getrennt werden können. Für die Anwendung der Verbindungen als Bioregulatoren ist jedoch eine Trennung der Enantiomeren oder Diastereomeren normalerweise nicht erforderlich. Die Erfindung umfaßt sowohl die Gemische als auch die optisch aktiven einheitlichen Substanzen.

Der Phenylrest kann unsubstituiert oder beispielsweise durch mindestens einen der Reste 2-Fluor, 4-Fluor-, 2-Chlor-, 3-Chlor-, 4-Chlor-, 4-Brom-, 2,4-Dichlor-, 2,4,6-Trichlor, 2-Methyl, 3-Methyl, 4-Methyl, 4-n-Propyl, 4-tert.-Butyl, 4-n-Butyl, 3-Trifluormethyl, 4-Trifluormethyl, 4-Phenyl, 4-Methoxy, 4-Butoxy, 4-Phe-

noxy, Naphthyl substituiert sein.

$R^1$ bedeutet beispielsweise Methylcyclohexyl, 2-Methyl-Tetrahydropyran-2-yl, tert.-butyl, 5-Methyl-1,3-dioxan-5-yl oder den Rest

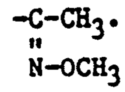

$R^2$ bedeutet beispielsweise Wasserstoff, Methyl, Ethyl, Propyl, Butyl oder Pentyl. Die Doppelbindung ist cis- oder vorzugsweise trans- konfiguriert.

$R^3$ bedeutet beispielsweise Wasserstoff, Vinyl, Allyl, Ethinyl oder Propinyl.

$R^4$ bedeutet beispielsweise Wasserstoff, Methyl, Ethyl, n-Propyl, Prop-2-enyl-(1), Prop-2-inyl-(1), n-Butyl, 2-Methylprop-2-enyl-(1), Acetyl, Propionyl, Butyryl, Isobutyryl.

Geeignete Säureadditionssalze sind beispielsweise die Bromide, Sulfate, Nitrate, Phosphate, Oxalate oder Dodecylbenzolsulfonate. Die Wirksamkeit der Salze geht auf das Kation zurück, so daß es auf das Anion i.a. nicht besonders ankommt.

Geeignete Metallkomplexe der Verbindungen (I) sind Verbindungen des Typs $Me[(I)_y]Q_x$, in der Me ein Äquivalent eines Metallions z. B. Kupfer, Zink, Zinn, Mangan, Eisen, Cobalt oder Nickel, Q ein Äquivalent eines Anions einer anorganischen Säure z.B. Salzsäure, Schwefelsäure, Phosphorsäure oder Bromwasserstoffsäure bedeutet und x und y für die zum Ausgleich der Wertigkeiten erforderlichen Zahlen stehen.

Die Verbindungen (I) werden im allgemeinen auf bekannte Art und Weise dargestellt; einige Herstellbeispiele sind weiter unten aufgeführt. Soweit es sich bei den Verbindungen (I) nicht um Ketone handelt, können diese sämtlich als Reduktionsprodukte von Ketonen, nämlich als die entsprechenden sekundären Alkohole und deren Veretherungs- oder Veresterungsprodukte angesehen werden.

Die unter die Formel I fallenden Ketone lassen sich ihrerseits herstellen, indem man andere Ketone, z.B. solche, die aus der DE-OS 3 019 049 oder DE-OS 2 638 470 bekannt sind, oder deren Alkalienolate mit entsprechenden 3-Arylallylhalogeniden in üblicher Weise alkyliert.

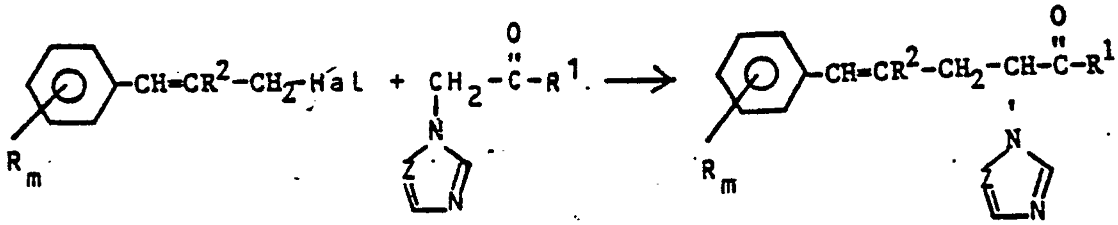
(Ia)

Man metalliert zunächst zu den Alkalienolaten, indem man vorzugsweise in Gegenwart eines polaren aprotischen Lösungsmittels wie Dimethylformamid oder Tetrahydrofuran mit einem Metallierungsagens wie Natriumhydrid, Lithiumdiisopropylamid oder n-Butyllithium umsetzt. Nach Zugabe des jeweiligen 3-Arylallylhalogenids erhält man das gewünschte Keton.

Die Azolverbindungen der Formel I, die durch $R^3$ = Wasserstoff dargestellt sind, werden vorzugsweise durch stereoselektive Reduktion der entsprechenden Ketone (I) und anschließende Alkylierung oder Acylierung hergestellt.

Dabei entstehen bei der Reduktion mit komplexen Hydriden, vorzugsweise mit Natriumborhydrid in einem protischen Verdünnungsmittel, vorzugsweise Methanol, Ethanol oder i-Propanol, diastereomere Alkohole einer bestimmten Konfiguration. Man erhält vorwiegend die jeweils anderen diastereomeren Alkohole, wenn man die Ketone mit einem sekundären Alkoholat oder mit Alkylmagnesiumhalogenid oder mit Wasserstoff katalytisch reduziert.

Verbindungen - hier Alkohole - mit zwei verschiedenen chiralen Kohlenstoffatomen bilden vier Stereoisomere, deren Konfiguration nach dem Cahn-Ingold-Prelog-System bezeichnet werden kann (siehe z. B. D. Seebach und V. Prelog, Angew. Chem. 94 , 696 (1982) und dort angegebene Literatur). Hierbei werden zwei Enantiomere der Konfigurationen R, R und S, S als Diastereomeres R*, R* bezeichnet.

Das andere Diastereomere mit der Bezeichnung R*, S* setzt sich aus den beiden Enantiomeren R, S und S, R zusammen.

Alkohole der Formel I mit Y = CHOH enthalten einen erheblich höheren Anteil mit R*, S*-Konfiguration als mit R*, R*-Konfiguration. Der Anteil der R*, R*-Diastereomeren im Gemisch liegt in der Regel weit unter 30 %. Reine R*, S*-Diastereomere können daraus durch einfaches Waschen der Rohprodukte mit geeigneten Lösungsmitteln. wie z.B. Diisopropylether, oder durch Umkristallisieren oder durch andere, übliche

Reinigungsschritte, z.B. Chromatographie erhalten werden.

Beispiel 1

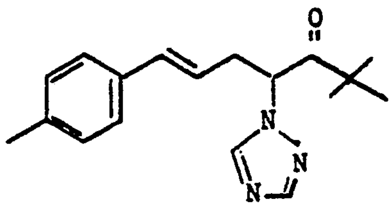

2,2,-Dimethyl-4-(1,2,4-triazol-1-yl)-7-(4-methylphenyl)-hepten-(6)-on-3

Zu 6,13 g (0,255 mol) Natriumhydrid in 100 ml DMF werden 42 g (0,251 mol) Triazolylpinakolon gelöst in 100 ml DMF zugetropft.

Nachdem die exotherme Reaktion abgeklungen ist, werden 41,63 g (0,25 mol) 3-(4-Methylphenyl)-allylchlorid zugetropft und über Nacht bei Raumtemperatur gerührt.

Nach Extraktion mit Wasser und Methylenchlorid wird die organische Phase über Natriumsulfat getrocknet.

Nach Abdampfen des Lösungsmittels erhält man in 86 %iger Ausbeute 64 g eines Öls, das durch folgende physikalischen Eigenschaften gekennzeichnet ist:

Infrarotabsorption bei
2969, 1717, 1513, 1502, 1478, 1275, 1138, 971, 679 nm.

Kernresonanzspektrum:
$^1$H-NMR (TMS, $CDCl_3$, ppm)
7,9 und 8,3 (je 1H, s)
7,0 - 7,2 (4H, m)
5,68 (1H, t), 5,9 (1H, m), 6,4 (1H, d)
2,3 (3H, s)
1,18 (9H, s)

Beispiel 2, 3

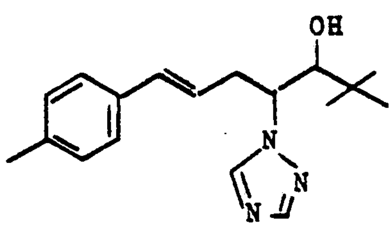

2,2-Dimethyl-4-(1,2,4-triazol-1-yl)-7-(4-methylphenyl)-hepten-(6)-ol-(3)

A) 28 g (0,094 mol) des nach Beispiel 1 erhaltenen Ketons werden in 200 ml Methanol durch portionsweise Zugabe von 4,3 g (0,113 mol) Natriumborhydrid reduziert. Man rührt 5 Stunden bei Rückfluß, dampft in Vakuum ein und nimmt mit Ether/Wasser auf. Nach zweimaliger Extraktion mit Ether, trocknet man die organische Phase über Natriumsulfat und dampft das organische Lösungsmittel ein.

Auf diese Weise erhält man 17 g ( 61 % Ausbeute) des einen diastereomeren Alkohols (A; Anteil des

diastereomeren Alkohols B nach $^1$H-NMR etwa 10 %; Wirkstoffbeispiel 2).

IR-Absorption bei 2956, 2871, 1512, 1276, 1135, 1096, 1070, 1018, 968, 979 nm.

Kernresonanzspektrum:
$^1$H-NMR ($CDCl_3$, TMS, ppm)
8,15 und 7,95 (je 1H, s)
7,0 - 7,2 (4H, m)
6,3 (1H, d)
5,9 (1H), m)
4,6 (1H, m)
2,8 und 2,9 (je 1H, m)
2,3 (3H, s)
0,65 (9H, s)

B) Aus n-Propylbromid und Mg werden in 100 ml Diethylether auf übliche Weise 0,1 mol n-Propylmagnesiumbromid erzeugt. 12,3 g (0,05 mol) des Ketons aus Beispiel 1 werden in 100 ml Diethylether zugetropft. Nach 5 Stunden Erhitzen am Rückfluß setzt man 200 ml wäßrige Ammoniumchloridlösung zu, trennt die Phasen und befreit die mit Natriumsulfat getrocknete organische Phase im Vakuum vom Lösungsmittel.

Auf diese Weise erhält man 13 g (86 % Ausbeute) des anderen diastereomeren Alkohols (B) als gelbes Öl. Anteil des diastereomeren Alkohols A etwa 5 %; Wirkstoffbeispiel 3.

IR-Absorption bei 2956, 2870, 1512, 1276, 1138, 1068, 1015, 968, 797, 679 nm.

Kernresonanzspektrum:
$^1$H-NMR ($CDCl_3$, TMS, ppm)
8,05 und 7,95 (je 1H, s)
6,95 - 7,25 (4H, m)
6,2 (1H, m)
5,8 (1H, m)
4,5 (1H, m)
3,7 (1H, m)
2,8 (2H, m)
2,3 (3H, s)
0,95 (9H, s)

Nach den vorstehenden Angaben werden die in der nachfolgenden Tabelle aufgeführten Verbindungen hergestellt soweit sie durch physikalische Angaben gekennzeichnet sind. Die nicht gekennzeichneten Verbindungen können durch entsprechende Abwandlung der Ausgangsstoffe und Umsetzungsbedingungen erhalten werden; sie lassen eine vergleichbare biologische Wirkung erwarten.

Tabelle

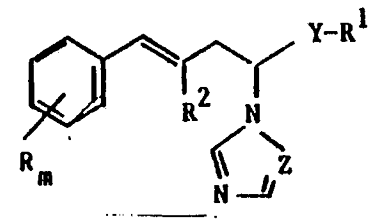

| Nr. | $R_m$ | $R^2$ | Z | Y | $R^1$ | Stereoform Typ | Fp | IR-Spektrum |
|---|---|---|---|---|---|---|---|---|
| 1 | 4-$CH_3$ | H | N | CO | $C(CH_3)_3$ | - | | |
| 2 | 4-$CH_3$ | H | N | CHOH | $C(CH_3)_3$ | A | | Wirkstoffbsp.1,2,3 |
| 3 | 4-$CH_3$ | H | N | CHOH | $C(CH_3)_3$ | B | | |
| 4 | H | H | N | CHOH | $C(CH_3)_3$ | A | 98-100°C | |
| 5 | 4-$OCH_3$ | H | N | CO | $C(CH_3)_3$ | - | Öl | 2568, 1716, 1608, 1512, 1465, 1276, 1245, 1176, 1033, 678 |
| 6 | 4-$OCH_3$ | H | N | CHOH | $C(CH_3)_3$ | A | Öl | 2556, 1708, 1511, 1288, 1277, 1248, 1176, 1135, 1034, 967 |

| Nr. | $R_m$ | $R^2$ | Z | Y | $R^1$ | Stereoform Typ | Fp | IR-Spektrum |
|---|---|---|---|---|---|---|---|---|
| 7 | 4-$C(CH_3)_3$ | H | N | CO | $C(CH_3)_3$ | - | Öl | 2964, 2905, 1716, 1502, 1477, 1366, 1275, 1138, 970, 678 |
| 8 | 4-$C(CH_3)_3$ | H | N | CHOH | $C(CH_3)_3$ | A | 134-136°C | |
| 9 | 4-Cl | H | N | CO | $C(CH_3)_3$ | - | 78-80°C | |
| 10 | 4-Cl | H | N | CHOH | $C(CH_3)_3$ | A | 118-120°C | |
| 11 | 4-Br | H | N | CO | $C(CH_3)_3$ | - | 68-70°C | |
| 12 | 4-Br | H | N | CHOH | $C(CH_3)_3$ | A | 130-133°C | |
| 13 | 4-Cl | H | N | CO | 1,3-dioxan--5-methyl-5-yl | - | Öl | |
| 14 | 4-Cl | H | N | CHOH | 1,3-dioxan--5-methyl-5-yl | A | 186-188°C | |
| 15 | 4-Cl | H | N | CHOH | 1,3-dioxan--5-methyl-5-yl | B | Öl | 2854, 1491, 1165, 1092, 1069, 1033, 1013, 969, 935, 926 |

| Nr. | $R_m$ | $R^2$ | Z | Y | $R^1$ | Stereoform Typ | Fp | IR-Spektrum |
|---|---|---|---|---|---|---|---|---|
| 16 | H | H | N | CO | 1,3-dioxan--5-methyl-5-yl | - | 111-113°C | |
| 17 | H | H | N | CHOH | 1,3-dioxan--5-methyl-5-yl | A | 147-150°C | |
| 18 | H | H | N | CHOH | 1,3-dioxan--5-methyl-5-yl | B | 81 | 1165, 1139, 1089, 1065, 1033, 567, 534, 926, 747, 684 |
| 19 | H | $C_5H_{11}$ | N | CO | 1,3-dioxan--5-methyl-5-yl | - | 72-74°C | |
| 20 | H | $C_5H_{11}$ | N | CHOH | 1,3-dioxan--5-methyl-5-yl | A | 100-103°C | |
| 21 | H | H | N | CO | Methylcyclohexyl | - | Harz | 2933, 1713, 1501, 1447, 1276, 1138, 967, 745 693, 678 |
| 22 | H | H | N | CHOH | Methylcyclohexyl | A | Harz | 2926, 2857, 1505, 1276, 1134, 1069, 964, 744, 692, 679 |

| Nr. | $R_m$ | $R^2$ | Z | Y | $R^1$ | Stereoform Typ | Fp | IR-Spektrum |
|---|---|---|---|---|---|---|---|---|
| 23 | H | H | N | CO | 2-Methyltetra-hydropyran-2-yl | - | Harz | 2541, 1729, 1502, 1276, 1138, 1080, 1047, 967, 746, 679 |
| 24 | H | H | N | CHOH | 2-Methyltetra-hydropyran-2-yl | A | Harz | 2938, 2864, 1502, 1276, 1136, 1106, 1079, 1049, 966, 693 |
| 25 | H | H | N | CO | $CH_3-C=N-O-CH_3$ | - | 78-80°C | |
| 26 | H | H | N | =NOH | $CH_3-C=N-O-CH_3$ | - | 188-191°C | |
| 27 | H | H | N | CHO-CO-$C_6H_4(2CO_2H)$ | tert.-butyl | A | Öl | |
| 28 | 4-$OCH_3$ | H | N | CHO-CO--$CH_2$-$CH_2$-$CO_2H$ | tert.-butyl | A | Öl | |
| 29 | 4-tert. | H | N | CH-O-CO-$C_6H_4(2CO_2H)$ | tert.-butyl | A | Öl | |

Wirkungsbeispiele

Als Vergleichssubstanz diente jeweils Chlorcholinchlorid (CCC).

Beispiel 1

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Dabei ergab sich, daß in Versuchen mit Sommergerste, Sommerweizen, Sonnenblumen und Sommerraps hinsichtlich Standfestigkeit und Wuchshöhenreduzierung bei Aufwandmengen von 1,5 und 6 mg Wirkstoff je Gefäß die Verbindungen 1 bis 6, 10, 17, 18, 21 und 22 durchweg eine günstigere Wirkung als der Vergleich erzielten.

Beispiel 2 (Reiskeimlingstest)

Junge Reiskeimlinge (Sorte Girona) wurden in einer Nährlösung kultiviert, die die jeweiligen Wirkstoffe in unterschiedlichen Konzentrationen enthielt. Nach sechs Tagen Kultur bei 25 °C im Dauerlicht wurde die Wirkstoffkonzentration bestimmt, die das Längenwachstum der zweiten Blattscheide um 50 % vermindert ($=KI_{50}$).
(Details bei: W. Rademacher und J. Jung, Berichte aus dem Fachgebiet Herbologie, Heft 24, pp 127-134, Universität Hohenheim, 1983)

Als Testsubstanz erzielte CCC einen Wert für $KI_{50}$ (molar) von $1{,}5 \times 10^{-2}$, die Verbindungen 1, 2, 3, 4, 6, 7, 8, 9, 12, 13, 15, 17, 18, 19, 20, 21, 22, 23, 24, 26, 28 und 29 erreichten Werte für $KI_{50}$ zwischen $1{,}8 \times 10^{-4}$ und $5 \times 10^{-6}$.

Beispiel 3 Saatgutbehandlung bei Sommergerste

Saatgut von Gerste (Sorte Aramir) wurde mit den jeweiligen Wirkstoffen beladen. Dazu wurden acetonische Wirkstofflösungen mit entsprechenden Mengen an Saatgut versetzt und am Vakuumrotationsverdampfer bei 30 °C bis zur Trockne eingeengt. Die Aufwandmenge lag dabei - je nach Molekulargewicht - im Bereich von 0,5 Gramm pro Kilogramm Saatgut. (Der genaue Wert in mg/kg ergibt sich durch Multiplikation des Molekulargewichts mit dem Faktor 2,35.) Die Kultivation der Pflanzen erfolgte unter Gewächshausbedingungen in Kunststoffgefäßen von 12,5 cm Durchmesser. Dem verwendeten Kultursubstrat wurden ausreichend Nährstoffe zugesetzt. Nach 12 Tagen wurden die erreichten Sproßlängen in % der unbehandelten Kontrollpflanzen erfaßt. In diesem Versuch erzielten die Verbindungen der Beispiele 1 und 3 bis 6 eine günstigere Wirkung als das zum Vergleich gewählte Chlorcholinchlorid.

## Ansprüche

1. Azolverbindungen der Formel I

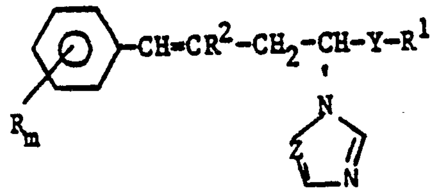

(I),

in welcher

R Fluor, Chlor, Brom, $C_1$-$C_4$-Alkyl, $C_1$-$C_4$-Alkoxy, Trifluormethyl, Phenyl oder Phenoxy bedeutet und m = 0 oder eine ganze Zahl von 1 bis 5 und wobei die einzelnen Atome oder Gruppen gleich oder verschieden sein können, wenn m größer als 1 ist,

$R^1$ tert.-Butyl, 5-Methyl-1,3-dioxan-5-yl, 1-Methylcyclohexyl, 2-Methyltetrahydropyran-2-yl oder

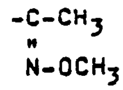

,

$R^2$ Wasserstoff oder $C_1$-$C_5$-Alkyl, ferner

Z> CH oder >N ist und >

Y >C=O, >C=N-OH oder >$CR^3OR^4$ bedeutet, mit der Maßgabe, daß nicht gleichzeitig Y = >C=O, $R^1$ = tert.-Butyl und m = 0 ist, und wobei

$R^3$ Wasserstoff, Methyl, Vinyl, Allyl, Ethinyl oder Propinyl und

$R^4$ Wasserstoff, $C_1$-$C_4$-Alkyl, $C_2$-$C_4$-Alkenyl, $C_2$-$C_4$-Alkinyl oder $C_2$-$C_4$-Alkanoyl bedeutet.

2. Verbindungen (I) gemäß Anspruch 1 in Form ihrer für Pflanzen verträglichen Säureadditionssalze und Metall-Komplexe.

3. Verfahren zur Herstellung der Azolverbindungen der Formel I gemäß Anspruch 1, bei denen $R^3$ = Wasserstoff ist, dadurch gekennzeichnet , daß man ein entsprechendes Keton der Formel II

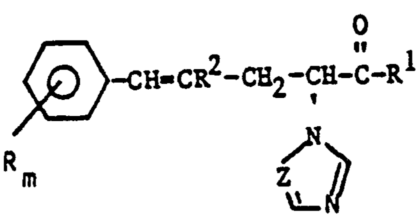

(II),

reduziert und den erhaltenen Alkohol entweder mit einem entsprechenden Alkanoylhalogenid oder -anhydrid oder mit einem entsprechenden Alkylierungsmittel umsetzt.

4. Verfahren zur Herstellung von Azolverbindungen der Formel I gemäß Anspruch 1 bzw. 2, bei denen $R^3$ = Methyl, Vinyl, Allyl, Ethinyl oder Propinyl ist, dadurch gekennzeichnet , daß man ein entsprechendes Keton der Formel II gemäß Anspruch 2 mit einer den Substituenten $R^3$ einführenden Grignardverbindung umsetzt, das erhaltene Alkoholat hydrolysiert und den erhaltenen tertiären Alkohol entweder mit einem Alkanoylhalogenid oder -anhydrid oder mit einem Alkylierungsmittel umsetzt.

5. Mittel zur Regulierung des Pflanzenwachstums, enthaltend einen Wirkstoff gemäß Anspruch 1.

6. Mittel zur Regulierung des Pflanzenwachstums, enthaltend einen Wirkstoff gemäß Anspruch 1 und einen festen oder flüssigen Trägerstoff.

7. Verfahren zur Regulierung des Pflanzenwachstums, dadurch gekennzeichnet , daß man eine Verbindung der Formel I gemäß Anspruch 1 auf Pflanzen, deren Lebensraum oder auf Saatgut einwirken läßt.

8. Verfahren zur Herstellung von Mitteln zur Regulierung des Pflanzenwachstums, dadurch gekennzeichnet , daß man eine Verbindung der Formel I gemäß Anspruch 1 mit festen oder flüssigen Trägerstoffen

mischt.

## Claims

1. An azole compound of the formula I

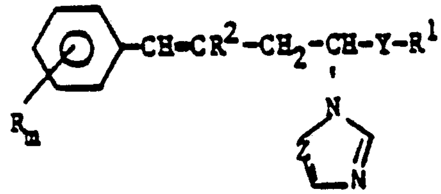

(I)

where R is fluorine, chlorine, bromine, $C_1$-$C_4$-alkyl, $C_1$-$C_4$-alkoxy, trifluoromethyl, phenyl or phenoxy and m is 0 or an integer from 1 to 5 and, when m is greater than 1, the individual atoms or groups may be identical or different, $R^1$ is tert-butyl, 5-methyl-1,3-dioxan-5-yl, 1-methylcyclohexyl, 2-methyltetrahydropyran-2-yl or

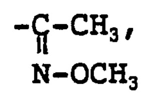

$R^2$ is hydrogen or $C_1$-$C_5$-alkyl, Y is $>C=O$, $>C=N\text{-}OH$ or $>CR^3OR^4$ with the proviso that Y is not $>C=O$ at the same time as $R^1$ is tert-butyl and m is O, and Z is $>CH$ or $>N$, $R^3$ is hydrogen, methyl, vinyl, allyl, ethynyl or propynyl and $R^4$ is hydrogen, $C_1$-$C_4$-alkyl, $C_2$-$C_4$-alkenyl, $C_2$-$C_4$-alkynyl or $C_2$-$C_4$-alkanoyl.

2. A compound (I) as claimed in claim 1, in the form of its plant-tolerated acid addition salts and metal complexes.

3. A process for the preparation of an azole compound of the formula I as claimed in claim 1, in which $R^3$ is hydrogen, wherein an appropriate ketone of the formula II

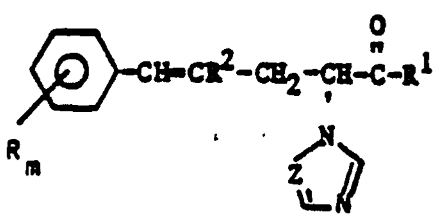

(II)

is reduced and the resulting alcohol is reacted either with an appropriate alkanoyl halide or anhydride or with an appropriate alkylating agent.

4. A process for the preparation of an azole compound of the formula I as claimed in claim 1 or 2, in which $R^3$ is methyl, vinyl, allyl, ethynyl or propynyl, wherein an appropriate ketone of the formula II according to claim 2 is reacted with a Grignard compound which introduces the substituent $R^3$, the resulting alcoholate is hydrolyzed and the tertiary alcohol obtained is reacted either with an alkanoyl halide or anhydride or with an alkylating agent.

5. A plant growth regulator, containing an active ingredient as claimed in claim 1.

6. A plant growth regulator, containing an active ingredient as claimed in claim 1 and a solid or liquid carrier.

7. A method of regulating plant growth, wherein a compound of the formula I as claimed in claim 1 is allowed to act on plants, on their habitat or on seed.

8. A process for the preparation of a plant growth regulator, wherein a compound of the formula I as claimed in claim 1 is mixed with a solid or liquid carrier.

## Revendications

1. Dérivés d'azole de formule

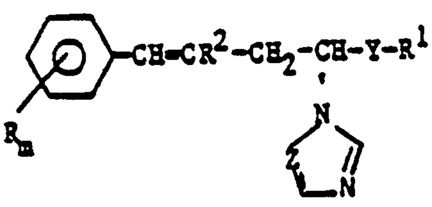

(I),

dans laquelle

R représente fluor, chlore, brome, alkyle en $C_1$-$C_4$, alcoxy en $C_1$-$C_4$, trifluorométhy1e, phényle ou phénoxy et m = 0 ou un nombre entier de 1 à 5, les différents atomes ou groupes pouvant être identiques ou différents si m est supérieur à 1,

$R^1$, tert-butyle, 5-méthyl-1,3-dioxan-5-yle, 1-méthyl-cyclohexyle, 2-méthyltétrahydropyran-2-yle ou

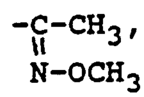

$R^2$, hydrogène ou alkyle en $C_1$-$c_5$, en outre

Z, ≥CH ou ≥N et

Y, ≥C = O, ≥C = N-OH ou ≥$CR^3OR^4$, étant entendu que Y, $R^1$ et m ne désignent pas simultanément : Y = ≥C = O, $R^1$ = tert-butyle et m = O et

$R^3$, représente hydrogène, méthyle, vinyle, allyle, éthynyle ou propynyle et

$R^4$, hydrogène, alkyle en $C_1$-$C_4$, alcényle en $C_2$-$C_4$, alcynyle en $C_2$-$C_4$ ou alcanoyle en $C_2$-$C_4$.

2. Composés (I), selon la revendication 1, sous forme de leurs sels d'addition d'acide et complexes métalliques tolérés par les plantes.

3. Procédé de préparation des dérivés d'azole de formule I, selon la revendication 1, dans lesquels $R^3$ = hydrogène, caractérisé par le fait que l'on réduit une cétone appropriée de formule II

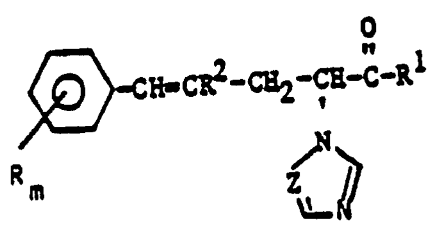

(II),

et on met à réagir l'alcool ainsi obtenu, soit avec un halogénure ou anhydride d'alcanoyle appropriés soit avec un agent d'alkylation approprié.

4. Procédé de préparation de dérivés d'azole de formule I, selon les revendications 1 ou 2, dans lesquels $R^3$ = méthyle, vinyle, allyle, éthynyle ou propynyle, caractérisé par le fait que l'on fait réagir une cétone appropriée de formule II, selon la revendication 2, avec un composé de Grignard introduisant le substituant $R^3$, on hydrolyse l'alcoolate ainsi obtenu et on met à réagir l'alcool tertiaire résultant, soit avec un halogénure ou anhydride d'alcanoyle soit avec un agent d'alkylation.

5. Agents pour régulariser la croissance des plantes, contenant un principe actif selon la revendication 1.

**6.** Agents pour régulariser la croissance des plantes, contenant un principe actif selon la revendication 1 et un véhicule solide ou liquide.

**7.** Procédé pour régulariser la croissance des plantes, caractérisé par le fait que l'on fait agir un composé de formule I, selon la revendication 1, sur les plantes, leur biotope ou les semences.

**8.** Procédé de préparation d'agents pour régulariser la croissance des plantes, caractérisé par le fait que l'on mélange un composé de formule I, selon la revendication 1, avec des supports solides ou liquides.